# EUROPEAN PATENT APPLICATION

(11) **EP 2 458 520 A1**
(43) Date of publication of application: **30.05.2012**
(21) Application number: 10015091.1
(22) Date of filing: 29.11.2010
(51) Int. Cl.: G06F 19/28

(54) **System for generating genetic information and corresponding method**

(71) Applicant: Lucic, Ivan, 1200 Vienna (AT)
(72) Inventor: Lucic, Ivan, 1200 Vienna (AT)
(74) Representative: Müller - Hoffmann & Partner

(57) **Abstract**

A system for generating a genetic information is provided, including a sensor for detecting a genetic sequence of a probe; a communication apparatus; and a database; wherein the sensor is adapted to the communicate the detected genetic sequence to the communication apparatus and the communication apparatus is adapted to communicate the detected genetic sequence to the database, and wherein the database is adapted to generate a genetic information based on the detected genetic sequence. A corresponding method is provided as well.

## Description

The invention relates to a system for generating a genetic information and a corresponding method.

### BACKGROUND

Deoxyribonucleic acid (DNA) is a nucleic acid that contains the genetic instructions used in the development and functioning of all known living organisms with the exception of some viruses. The main role of DNA molecules is the long-term storage of information. DNA is often compared to a set of blueprints, like a recipe or a code, since it contains the instructions needed to construct other components of cells, such as proteins and RNA (ribonucleic acid) molecules. In recent times possibilities for analyzing probes in order to derive a genetic sequence are increasing. However, until now the use of genetic sequences is restricted to clinical environments, e.g. hospitals or laboratories.

Thus, there is a need for providing a system and a method for generating a genetic information and/or enabling further uses of genetic sequences. This need is arising especially in the area of health care in geographical regions with low infrastructure but in other profane usage as well.

In the following, genetic information is a term referring to any one of DNA-information, RNA-information or protein information and genetic sequence is a term referring to any one of DNA-sequence, RNA-sequence or amino-acid sequence.

The object is solved by a system for generating a genetic information according to claim 1, a corresponding method according to claim 20 and a further system according to claim 22.

Further embodiments are defined in the dependent claims.

Further details of the invention will become apparent from the consideration of the drawings and ensuing description.

### BRIEF DESCRIPTION OF THE DRAWINGS

- Fig. 1: shows a schematic block diagram of a system according to an embodiment of the invention,
- Fig. 2: shows a schematic block diagram of a system according to a further embodiment of the invention, and
- Fig. 3: shows schematically steps of a method according to an embodiment of the invention.

### DETAILED DESCRIPTION

In the following, embodiments of the invention are described. It is important to note that all described embodiments in the following and their properties and technical features may be combined in any way, i.e. there is no limitation that certain described embodiments, properties and technical features may not be combined with others.

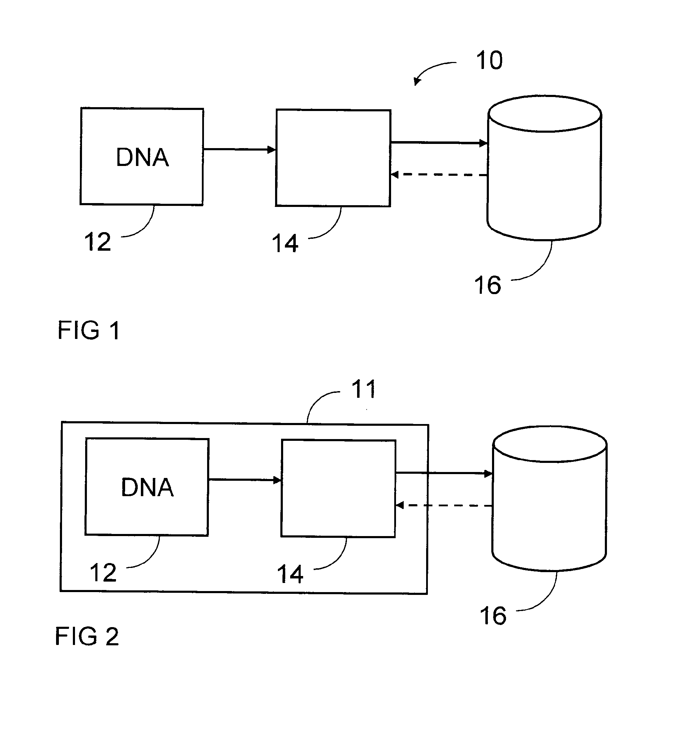

In Fig. 1 a system 10 for generating a genetic information is depicted, which comprises a sensor 12 for detecting a genetic sequence of a probe, a communication apparatus 14 and a database 16. The sensor 12 is adapted to communicate the detected genetic sequence to the communication apparatus 14, which is adapted to communicate the detected genetic sequence to the database 16 and the database 16 is adapted to generate a genetic information based on the detected genetic sequence.

The term "generating a genetic information" is used herein to cover as well "verifying" or "justifying" a genetic information, i.e. checking whether a detected genetic sequence of a probe corresponds to an expected genetic sequence of this particular probe.

According to a further embodiment it is possible that the database 16 is further adapted to transmit the genetic information to the communication apparatus 14 and the communication apparatus 14 is adapted to receive the genetic information.

If the database 16 is realized as a remote database, the remote database might be updated easier and might include more data than a database that might be realized within the sensor 12 or within the communication apparatus 14. The remote database might include a memory and a processor, wherein the processor is adapted to generate the genetic information based on the transmitted genetic sequence.

The remote database might include the storage and the processing of received data. The remote database might include a possibility for global estimation of different assets according to the received data like global funds, foundations, movement of legal and real-estate assets, hedge funds but also other than economical asset values like social, demographic and movement of goods as well as estimation of public interests and collective values. The remote database might be used for defining global and/or regional geographical patterns of disease spread and control. The remote database might include collecting and processing of received data for environmental purposes. The remote database might include collecting and processing of received data for scientific purposes. The remote database might include collecting and processing of received data for economical and business purposes. The remote database might be used for global and/or regional statistic purposes. The remote database might be used as a data source for different analysis like: medical (human, plant or animal), technical, economical, nutritional, judicial, forensic, life-style, fun or similar.

If the database 16 is realized as a memory and a processor within the sensor 12 or within the communication apparatus 14, however, the genetic information might be delivered faster and even in case without a connection to an external network.

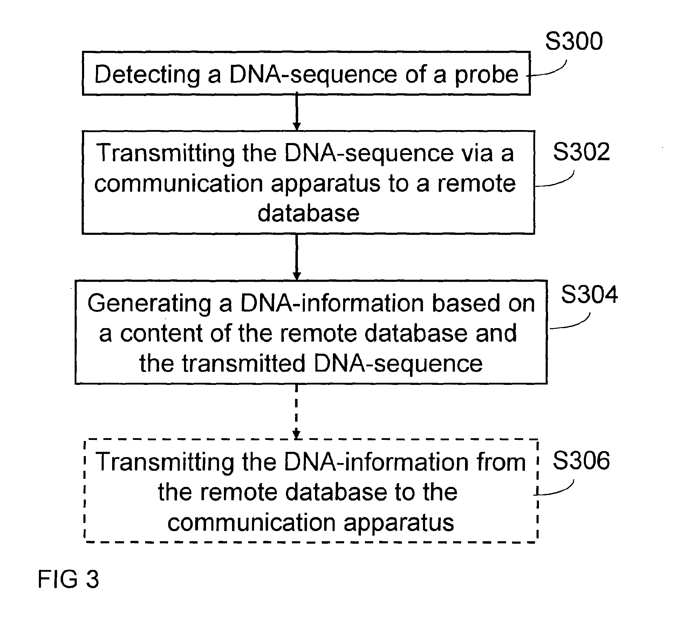

According to Fig. 3, the method for generating a genetic information comprises a step S300, wherein a genetic sequence of a probe is detected, a step S302 wherein the genetic sequence is transmitted via a communication apparatus to a database" e.g. a remote database, a step S304 wherein a genetic information is generated based on a content of the database and the transmitted genetic sequence and optionally a step S306 wherein the genetic information is transmitted from the database to the communication apparatus.

The sensor 12 might be realized as an in-vitro diagnostic-platform (IVD) based on a micro-fluidic chip which includes a plurality of different sensors that are built with a plurality of different reagents, buffers, enzymes or other specific or nonspecific chemicals that can perform and/or are needed for performance of different assays and is able to provide a genetic sequence of the probe. For very simple organisms the term "genetic sequence" might refer to the complete genetic sequence, whereas it might also refer to parts of the complete genetic sequence of more complex organism.

The sensor 12 is connected to a communication apparatus 14, e.g. by a wire-based connection or the wireless-based connection, e.g. by USB (Universal Serial Bus) or Bluetooth®. The communication apparatus 14 might be realized as a the cellular phone, a personal digital assistant, a laptop with communication abilities, a smart phone or a similar communication device.

The sensor 12 might be included in a read-out box connectable with the cellular phone.

According to a further embodiment depicted in Fig. 2 it is possible that the sensor 12 and the communication apparatus 14 are integrated in a further system 11 that is' enabled to detect the genetic sequence and to transmit it to the database 16, e.g. a remote database. If the communication apparatus 14 includes a camera, it might be possible to use the camera as a part of the sensor 12 in order to derive the DNA sequence. If the sensor 12 derives the genetic sequence based on electrochemical detection, the sensor 12 or the communication apparatus 14 might be adapted to perform an impedance measurement for deriving the genetic sequence. Further read-out possibilities include but are not restricted to: magnetic read-out due to the labeling with magnetic beads and due to the measurement of magnetic field changes; changes of mass (cantilever read-out method) or similar methods.

The further system might be realized as a cellular phone, a smart phone or the like with a connected or included sensor 14.

The genetic sequence might be saved in an internal memory of the sensor 12 or the communication apparatus 14. It is also possible to realize the database in the communication apparatus 14 or in the sensor 12, e.g. as a part of respective internal memories.

The communication apparatus 14 transmits the genetic sequence to a remote database 16 via any known network/protocol, e.g. via GPRS (General Packet Radio Service), UMTS (Universal Mobile Telecommunications System), WCDMA (Wide-band Code Division Multiple Access) or internet protocols like TCP/IP (Transmission Control Protocol/Internet Protocol), other current or future protocols or the like.

The result from the remote database 16, i.e. the genetic information, might be transmitted back to the communication apparatus 14 by SMS (Short Message Service), MMS (Multimedia Messaging Service) or any similar data format.

The genetic sequence and/or the genetic information might be transmitted in a non-encrypted version. It is also possible to transmit it in an encrypted version in order to protect private data and to conform with the Data Protection Act (DPA)

The database 16, e.g. remote database, might include a list of artificial body parts related to predetermined genetic sequences, and the database might be adapted to derive an artificial body built on the basis of the transmitted genetic sequence and transmit the artificial body as the genetic information to the communication apparatus 14. This embodiment might be used for gaming purposes, since it is possible to build avatar which is a computer user's representation of himself/herself in the form of a three-dimensional model used in computer games. In this embodiment it is possible to build an avatar based on a genetic sequence from the computer user.

In a further embodiment it is possible that the database 16, e.g. remote database, includes a list of achievable body fitness related to predetermined genetic sequences, and the database 16 generates the achievable body fitness based on the transmitted genetic sequence and transmit it as the genetic information to the communication apparatus 14. With this embodiment, a user might analyze his own genetic sequence and gets his achievable body fitness as a result to be displayed on his communication apparatus 14.

According to a further embodiment, the database 16, e.g. remote database, might include a list of different sounds related to predetermined genetic sequences and the database 16 might generate a tone that can be used as a ring-tone for a cellular phone as the genetic information based on the sound corresponding to the transmitted genetic sequence. In this embodiment the user might generate an individual ring-tone for his cellular or smart phone based on his own genetic sequence or might use the tone as music or for alternative therapies.

According to a further embodiment the database, e.g. remote database, might include a list of food data or other products or conglomerates data including living beings and/or their products that can be genetically modified, related to predetermined genetic sequences and the database 16 generates a DNA-analysis of food or other products as the genetic information. It might transmit the genetic information to the user of the communication apparatus 14, who knows afterwards whether e.g. the food or other products include genetically modified organisms or not.

According to a further embodiment the database 16, e.g. remote database, might include animal data related to predetermined genetic sequences and the database 16 generates a DNA-analysis of the animal in order to derive health-information or parentage information for using it, e.g. for breeding and selling animals.

According to a further embodiment the database 16, e.g. remote database, might include environmental data related to predetermined genetic sequences and the database 16 might generate the DNA-analysis of the environment as the genetic information. This might be used, e.g. for analyzing water or air quality.

According to a further embodiment, the database 16, e.g. remote database, might include species information related to predetermined genetic sequences, and the database 16 might generate an identified species as the genetic information. With this embodiment it is possible to identify the species of an animal, plant or a virus by simply analyzing the genetic sequence, transmitting it to the database 16 and receiving the identified species on the communication apparatus 14 afterwards.

According to a further embodiment the database 16, e.g. remote database, might include an encryption method and the database 16 generates an encrypted message based on the encryption method and the transmitted genetic sequence as the genetic information. This might be used for establishing a very secure encryption method, since it is only possible to encrypt it with the genetic sequence available to the user of the communication apparatus 14.

According to a further embodiment, the database 16, e.g. remote database, encloses a list of human individuals related to predetermined genetic sequences and the genetic information generated by the database is a name of the human individual with the transmitted genetic sequence. This might be used in forensic investigations, by immigration purposes or for safe entry in security areas.

According to a further embodiment the database 16, e.g. remote database, might include a list of products related to predetermined genetic sequences and the database 16 might generate an origin of the product based on the genetic sequence. This might be used in order to ensure that the product has been manufactured by the company which provided the product with the correct genetic sequence, respectively to prevent unauthorized imitations, copies and counterfeits.

According to a further embodiment, the database 16, e.g. remote database, might include a list of identification codes related to predetermined genetic sequences, and the generated genetic information is an identification code. This might be used for safe entry into security areas, for user identification in banking applications or for other identification purposes.

According to a further embodiment, the database 16, e.g. remote database, might include a list of pathologic or other human, plant or animal health-related genes related to predetermined genetic sequences and the database might generate a disease information based on the pathologic genes and transmit it to the communication apparatus 14. This might help in identifying diseases earlier by simply analyzing the genetic sequences and transmitting it to the database 16.

Instead of analyzing the probe and detecting a genetic sequence it might also be possible to derive e.g. the hormone level or perform any other biochemical analysis and transmit the results via a communication apparatus to the database 16.

With the disclosed embodiments it is possible to improve the time-to-answer, since the result is directly communicated from the communication apparatus to the database and is transmitted thereafter to the communication apparatus again. Further on, the read-out is simplified and the user interface is easier to handle. Existing optical chip technology of cell phones might be used for developing a simplistic read-out. Advantageously a cell phone controller might be used as controller for an DNA-test performance its processing data flow management and for record purposes. Advantageously an integrated micro fluidic chip based diagnostic device is integrated on a cellular phone or similar communication apparatus. Due to fitting the components of the system for large scale manufacturing and knowledge of broad agreement on concepts and specifications for standards, an aspect of the targeted product is its low cost production. The system provides a good sensitivity and specificity. It might be used by minimally trained personnel, because of the simplistic handling which means low volume sample (e.g. 2*µ*l), full-fledged automated sample processing and yes or no answer. The components are able to work at high temperatures and humidity and are able to store the data for long periods of time without refrigeration. Current on-shelf times are up to 2 years due to dried chemicals on-board. The system is also able to conduct tests without the need for local reagents/water and/or specialized laboratory equipment. In addition, it is possible to detect multiple pathogens or to distinguish between different pathogens and/or strains and subtypes. Multiple DNA/RNA-changes can be detected for different purposes.

## Claims

1. System for generating a genetic information comprising:
a sensor for detecting a genetic sequence of a probe;
a communication apparatus; and
a database;
wherein the sensor is adapted to communicate the detected genetic sequence to the communication apparatus and the communication apparatus is adapted to communicate the detected genetic sequence to the database, and wherein the database is adapted to generate a genetic information based on the detected genetic sequence.

2. System according to claim 1, wherein the database is further adapted to transmit the genetic information to the communication apparatus and the communication apparatus is adapted to receive the genetic information.

3. System according to any one of claims 1 or 2, wherein the communication apparatus is a cellular phone.

4. System according to any one of claims 1 to 3, wherein the sensor is chip-based.

5. System according to any one of claims 1 to 4, wherein the database is a remote database.

6. System according to any one of claims 1 to 5, wherein the database includes a list of artificial body parts related to predetermined genetic sequences, and the genetic information is an artificial body built on the basis of the transmitted genetic sequence.

7. System according to any one of claims 1 to 5, wherein the database includes a list of achievable body fitness related to predetermined genetic sequences, and the genetic information is the achievable body fitness based on the transmitted genetic sequence.

8. System according to any one of claims 1 to 5, wherein the database includes a list of different sounds related to predetermined genetic sequences, and the genetic information is a ring-tone based on a sound corresponding to the transmitted genetic sequence.

9. System according to any one of claims 1 to 5, wherein the database includes a list of food data related to predetermined genetic sequences, and the genetic information is a DNA-/RNA-/protein-analysis of the food.

10. System according to any one of claims 1 to 5, wherein the database includes animal data related to predetermined genetic sequences, and the genetic information is a DNA-/RNA-/protein-based analysis of animals.

11. System according to any one of claims 1 to 5, wherein the database includes environmental data related to predetermined genetic sequences, and the genetic information is a DNA-/RNA-/protein-based analysis of the environment.

12. System according to any one of claims 1 to 5, wherein the database includes species information related to predetermined genetic sequences, and the genetic information is an identified species.

13. System according to any one of claims 1 to 5, wherein the database includes plant data related to predetermined genetic sequences, and the genetic information a DNA-/RNA-/protein-based analysis of plants.

14. System according to any one of claims 1 to 5, wherein the database includes virus data related to predetermined genetic sequences, and the genetic information is a DNA-/RNA-/protein-based analysis of viruses.

15. System according to any one of claims 1 to 5, wherein the database includes an encryption method, and wherein the genetic information is an encrypted message based on the encryption method and the transmitted genetic sequence.

16. System according to any one of claims 1 to 5, wherein the database includes a list of human individuals related to predetermined genetic sequences, and the genetic information is a name of a human individual with the transmitted genetic sequence.

17. System according to any one of claims 1 to 5, wherein the database includes a list of products related to predetermined genetic sequences, and the genetic information is an origin of the product.

18. System according to any one of claims 1 to 5, wherein the database includes a list of identification codes related to predetermined genetic sequences, and the genetic information is an identification code.

19. System according to any one of claims 1 to 5, wherein the database is a list of pathologic genes related to predetermined genetic sequences, and the genetic information is a disease identification.

20. Method for generating a genetic information comprising:
detecting a genetic sequence of a probe;
transmitting the genetic sequence via a communication apparatus to a database;
generating a genetic information based on a content of the database and the transmitted genetic sequence.

21. Method for generating a genetic information according to claim 20, further comprising
transmitting the genetic information from the database to the communication apparatus.

22. System for generating a genetic information comprising:
a sensor for detecting a genetic sequence of a probe; and
a communication apparatus;
wherein the sensor is adapted to the communicate the detected genetic sequence to the communication apparatus and the communication apparatus is adapted to communicate the detected genetic sequence to a database.
